(19) **Europäisches Patentamt**

**European Patent Office**

**Office européen des brevets**

(11) **EP 0 995 762 A2**

(12) **EUROPEAN PATENT APPLICATION**

(43) Date of publication:
26.04.2000 Bulletin 2000/17

(51) Int Cl.⁷: **C08F 2/34**, C08F 2/46,
C08J 7/18, G02B 1/04

(21) Application number: 99308233.8

(22) Date of filing: 19.10.1999

(84) Designated Contracting States:
**AT BE CH CY DE DK ES FI FR GB GR IE IT LI LU MC NL PT SE**
Designated Extension States:
**AL LT LV MK RO SI**

(30) Priority: 20.10.1998 US 175165

(71) Applicant: **JOHNSON & JOHNSON VISION PRODUCTS, INC.**
**Jacksonville, Florida 32216 (US)**

(72) Inventor: **Jen, James S.**
**Jacksonville, FL 32256 (US)**

(74) Representative: **Mercer, Christopher Paul**
**Carpmaels & Ransford**
**43, Bloomsbury Square**
**London WC1A 2RA (GB)**

(54) **Coatings for biomedical Devices**

(57) The invention provides devices having gas-phase deposited coatings. Specifically, this invention provides devices, such as biomedical devices, with gas-phase deposited vinyl pyrrolidone, N, N'-dimethylacrlyamide, ethylene glycol, vinyl acetate, vinyl acetic acid, acrylic acid, and 3,3-dimethylacrylic acid coatings that are non-fouling and wettable.

## Description

Field of the Invention

This invention relates to devices having gas-phase deposited coatings. More specifically, this invention relates to devices, such as biomedical devices, with gas-phase deposited vinyl pyrrolidone, N, N'-dimethylacrlyamide, ethylene glycol, vinyl acetate, vinyl acetic acid, acrylic acid, and 3,3-dimethylacrylic acid coatings that are non-fouling and wettable.

Background of the Invention

The chemical composition of surfaces plays a pivotal role in dictating the overall efficacy of many devices. Some devices require non-fouling, and wettable surfaces in order for the devices to be useful for their intended purposes. For example, many biomedical devices such as catheters, stents, implants, intraocular lenses and contact lenses require surfaces that are biologically non-fouling, which means that proteins, lipids, and cells do not adhere to the surfaces of the devices. In some cases, materials for devices are developed that have all the necessary attributes for their intended purposes such as strength, optimal transmission, flexibility, stability, and gas transport except that the surfaces of the materials foul when in use. In these cases either new materials for the devices are developed or an attempt to change the surface characteristics of the materials is made.

In the specific case of contact or intraocular lenses, particularly contact lenses, although many polymeric materials possess the necessary mechanical, oxygen permeation and optical properties required for lens manufacture, many potential contact lens materials are subject to rapid fouling due to the adhesion of proteins, lipids. Additionally, the surface energies of the materials may be too low making the contact lenses too hydrophobic and, therefore, not wettable by the tear fluid.

The coating of biomedical devices using plasma treatment and deposition to alter surface characteristics has been disclosed. However, such methods have a number of disadvantages including complexity, problems with coating uniformity and depth, and stability. PCT/US90/05032 (Int. Publication No. W0 91/04283) discloses increasing the wettability of polymeric contact lens materials synthesized from specific hydroxy acrylic units and vinylic siloxane monomers by grafting other molecules to the surface. The only examples of the proposed grafting procedure described in this patent involve attachment of specific polyols by wet chemical procedures, but this patent does suggest that hydroxy acrylic units may be grafted to the specific hydroxy acrylic/siloxane polymeric materials by radiation methods.

U.S. Patent Nos. 3,854,982 and 3,916,033 describe the use of liquid coating techniques to improve the wettability of contact lens polymers. In these procedures free radical polymerizable precursors, including hydroxy alkyl methacrylates, are attached to contact lenses by exposure to high energy radiation. However, these solution attachment processes provide poor control of the film thickness and these films exhibit poor abrasion resistance, particularly when attached to polysilicone substrates.

The need still remains for a stable coating composition that can be uniformly applied and that can be applied to a substrate of a device to provide a non-fouling, and wettable, or hydrophilic, surface.

Description of the Invention and Preferred Embodiments

The invention provides biomedical devices with non-fouling coating compositions and processes for producing such devices. The coating compositions provide surfaces that are uniform, pin-hole free, wettable, devoid of extractables and chemically stable. Further, the coatings exhibit excellent optical transparency in the visible region of the electromagnetic spectrum, are oxygen permeable, and are abrasion resistant.

The present invention provides a device comprising, consisting essentially of, and consisting of at least one surface having a coating effective amount of a coating composition, said coating composition being formed by the gas phase polymerization of a gas comprising, consisting essentially of, and consisting of at least one monomer, said monomer selected from the group consisting of vinyl pyrrolidone, N, N'-dimethylacrylamide, ethylene glycol, vinyl acetate, vinyl acetic acid, acrylic acid, 3,3-dimethylacrylic acid, and mixtures thereof.

In another embodiment, the invention provides a method for manufacturing devices comprising, consisting essentially of, and consisting of contacting at least one surface of a device with a coating effective amount of a coating composition, said coating composition being formed by the gas phase polymerization of a gas comprising, consisting essentially of, and consisting of at least one monomer, said monomer selected from the group consisting vinyl pyrrolidone, N, N'-dimethylacrylamide, ethylene glycol, vinyl acetate, vinyl acetic acid, acrylic acid, 3,3-dimethylacrylic acid, and mixtures thereof.

Preferably, the device is a biomedical device. By "biomedical device" is meant a device designed to be used while in or on either or both human tissue or fluid. Examples of such devices include, without limitation, stents, implants, catheters, and ophthalmic lenses. In a more preferred embodiment, the biomedical device is an ophthalmic lens including, without limitation, contact or intraocular lenses. Most preferably, the device is a contact lens.

The devices of this invention may be made of any suitable material or materials such as polymers, ceramics, glass, silanized glass, fabrics, paper, metals, silanized metals, silicon, carbon, silicones hydrogels, and mixtures thereof. The more preferred materials are silicone and silicone containing compositions (mixed blends and copolymers), polyurethanes, and hydrogels, and mixtures of these materials. The most preferred materials are those polymers that do not support a stable tear film, such as silicones, silicone mixed blends, alkoxylated methyl glucosides, fluorinated hydrocarbons, silicone hydrogels, polyurethane-silicone hydrogels, fluorinated hydrocarbon hydrogels, polysulfones, and mixtures thereof.

Illustrative silicones include, without limitation, polydimethyl siloxane poly-dimethyl-co-vinylmethylsiloxane, silicone rubbers described in U.S. Patent No. 3,228,741, silicone blends such as those described in U.S. Patent No. 3,341,490, and silicone compositions such as described in U.S. Patent No. 3,518,324. Useful silicone materials include, without limitation, crosslinked polysiloxanes obtained by crosslinking siloxane prepolymers by means of hydrosilylation, co-condensation and by free radical mechanisms. Particularly suitable materials are organopolysiloxane polymer mixtures that readily undergo hydrosilylation. Such prepolymers contain vinyl radicals and hydride radicals that serve as crosslinking sites during chain extension and crosslinking reaction and are of the general formulation polydihydrocarbyl-co-vinylhydrocarbylsiloxane and polydihydrocarbyl-co-hydrocarbylhydrogensiloxanes wherein the hydrocarbyl radicals are monovalent hydrocarbon radicals such as alkyl radicals having 1-7 carbon atoms, including, without limitation, methyl, ethyl, propyl, butyl, pentyl, hexyl and heptyl; aryl radicals, such as phenyl, tolyl, xylyl, biphenyl; haloaryl, such as chlorophenyl and cycloalkyl radicals such as cyclopentyl, cyclohexyl, and the like.

The more preferred materials are silicone hydrogels, particularly silicone-hydrogels formed from monomer mixtures of an acrylic-capped polysiloxane prepolymer, a bulky polysiloxanylalkyl (meth)acrylate monomer and hydrophilic monomers as described in U. S. Patent Nos. 5,387,632; 5,358,995; 4,954,586; 5,023,305; 5,034,461; 4,343,927; and 4,780,515. Other preferred materials include cyclic polyols of alkoxylated glucose or sucrose like those described in U.S. Patent Nos. 5,196,458 and 5,304,584, and U.S. Patent Application Serial No. 08/712,657 filed September 13, 1996. All of the patents cited above are incorporated in their entireties herein by reference.

The coating compositions of the invention are formed from gas phase deposited monomers of vinyl pyrrolidone, N, N'-dimethylacrylamide, ethylene glycol, vinyl acetate, vinyl acetic acid, acrylic acid, 3,3-dimethylacrylic acid and mixtures thereof. The monomers are commercially available or may be synthesized by known methods. The coating compositions of the invention are stable and adhere to a wide range of materials. The weight average molecular weights of the monomers are preferably less than about 400, more preferably less than about 300, and most preferably less than about 200.

The coating compositions of the invention may be the result of the polymerization of substantially a single monomer or of a mixture of monomers including the addition of cross-linking agents. The mixture of monomers preferably are selected from the monomers described above. The preferred coating compositions are substantially a single monomer.

In the case in which the monomer used is ethylene glycol, the method of its application to a surface is selected so that the surface preferably is provided with a coating composition in which the outermost layer of the coating has a ratio of carbon-oxygen bonds to carbon-carbon bonds of greater than about 1:1, more preferably greater than about 1.5:1, and most preferably greater than about 2:1, even more preferred is greater than 2.5:1. The coating compositions having a higher ratio of carbon-oxygen bonds to carbon-carbon bonds are preferred, because of improved non-fouling and higher wettability characteristics.

The preferred method for depositing the coating compositions on the substrates is by gas phase deposition, because it provides uniform coating compositions. Gas phase deposition means by any method the gaseous monomers are attached to the solid substrate as a surface coating. Gas phase depositions include, without limitation, plasma and photochemical induced polymerizations. Plasma induced polymerizations or plasma depositions are polymerizations due to the generations of free radicals caused by passing an electrical discharge through a gas. The electrical discharge can be caused by high voltage methods, either alternating current ("AC") or direct current ("DC"), or by electromagnetic methods, such as, radio frequency ("RF") or microwave. Alternatively, the coating process can be carried out using photochemical induced polymerizations as provided by direct absorption of photons of sufficient energy to create free radicals and/or electronically excited species capable of initiation of the polymerization process.

The more preferred method of gas phase deposition is by plasma polymerization, particularly RF plasma polymerization. This process is fully described in U. S. Patent Application No. 08/632,935, incorporated herein in its entirety by reference. Additional descriptions can be found in Panchalingam et al., "Molecular Surface Tailoring of Biomaterials Via Pulsed RF Plasma Discharges," J.Biomater. Sci. Polymer Edn., Vol. 5, No. 1/2, pp. 131-145 (1993), and Panchalingam et al., "Molecular Tailoring of Surfaces Via Pulsed RF Plasma Depositions," J. App. Sci.: Applied Polymer Symposium 54, pp.123-141 (1994), incorporated herein in their entireties by reference.

In this method, coatings are deposited on solid surfaces via plasma polymerization of selected monomers under controlled conditions. The plasma is driven by RF radiation using coaxial or parallel internal or external RF electrodes located around the exterior or interior of a reactor. Surfaces to be coated are preferably located in the reactor between the RF electrodes; however, the surfaces can be located either before or after the electrodes. The reactor is evacuated to background pressure using a vacuum pump. A fine metering valve is opened to permit vapor of the coating composition to enter the reactor. The pressure and flow rate of the monomer through the reactor is controlled by adjustments of the metering valve and a butterfly control valve (connected to a pressure controller) located downstream of the reactor. In general, the monomer reactor pressures employed range from about 5 to 200 mili-torr, although values outside this range can also be utilized. It is preferred that the monomers have sufficiently high vapor pressures so that the monomers do not have to be heated above room temperature (20 to 25°C) to vaporize the monomers.

The chemical composition of a film obtained during plasma deposition is a strong function of the plasma variables employed, particularly the RF power used to initiate the polymerization processes. It is preferred to operate the plasma process under pulsed conditions, as compared to continuous wave ("CW"), because it is possible to employ reasonably large peak powers during the plasma on initiation step while maintaining a low average power over the course of the coating process. Pulsing means that the power to produce the plasma is turned on and off. The average power under pulsing is defined as:

$$\text{Average Power} = \frac{\text{plasma on time}}{\text{plasma (on + off) time}} \text{ X Peak Power}$$

For example, a plasma deposition carried out at a RF duty cycle of 10 ms on and 200 ms off and a peak power of 25 watts corresponds to an average power of only 1.2 watts. Generally, the duty cycle, the ratio of plasma on to plasma off times, is less than about 1/5, preferably about 1/10 to 1/1000, more preferably about 1/10 to 1/30. The plasma on times are generally larger than about 1 $\mu$sec, preferably about 10 $\mu$sec to 100 msec, more preferably about 100 $\mu$sec to 10 msec. Plasma off times generally are larger than about 4 $\mu$sec, preferably about 100 $\mu$sec to 2000 msec, more preferably about 200 $\mu$sec to 100 msec. The Peak Power is preferably between about 10 and 300 Watts.

Pulsed plasma deposition permits use of relatively high peak powers while simultaneously maintaining relatively low average powers which provides for the retention of monomer functional groups. Coating compositions deposited under low average power pulsed conditions tend to be more adhesive to a given substrate when compared to films deposited at the same average power but under CW operation. For a given average power, the momentary high peak power available under pulsed conditions apparently assists in obtaining a stronger grafting of the film to the substrate than that obtained under the same average power CW conditions.

For a given RF peak power, an increased retention of the ether content (C-O functionality) of the plasma generated coating is observed as the plasma duty cycle is reduced when working with a given monomer. Alternatively, the chemistry of the coating composition may be varied under pulsed conditions by working at a single plasma duty cycle, but varying peak powers. There is an increased incorporation of C-O functionality in coating compositions as the peak power is increased. The plasma generated film composition can be varied by changing the plasma on to plasma off pulse widths, at a fixed ratio of plasma on to plasma off times and at a fixed RF peak power.

The chemical composition of the films of this invention may be varied during pulsed plasma deposition, by varying the peak and average powers, the duration of the plasma on and plasma off pulse widths when working at a constant average power, and the average power. To produce a coating composition with the preferred ratio of C-O functionality to C-C functionality, it is preferred that the Average Power of the pulsed plasma deposition is less than about 100 Watts, more preferably less than about 40 Watts, most preferably less than about 10 Watts. The highest ratios of C-O functionality to C-C functionality can be obtained when the Average Power is about 1 Watt and less which provides the most non-fouling and wettable coating compositions; however, depending on the coating materials, typically when the coating materials are applied at an Average Power around 0.5 Watts or less, the adhesion of the coating composition to the surface may be too weak to be useful for any purpose.

The use of lower Average Power conditions increases the presence of functional groups, e.g. ether units, in the coatings, but the less energetic deposition conditions at lower average power results in poorer adhesion of the polymer film to the underlying substrate. Thus, the plasma coating process involves somewhat of a compromise between retention of monomer integrity in the plasma generated film and the strength of the adhesion between the coating and the solid substrate. In the case of contact lenses, the adhesion and overall stability of the coating composition to the lens substrate is an extremely important consideration.

A preferred method of applying the coating compositions to the surface is by gradient layering pulsed plasma deposition, which can be used to maximize the adhesion of the coating composition and the functionalities present in the coating composition. This method is described further in U. S. Patent Application Serial No. 08/632,935. In this process, an initial high power, high plasma duty cycle is employed to graft the plasma generated coating composition tightly to the underlying substrate. The plasma duty cycle is subsequently decreased in a systematic manner, with each decrease resulting in an increased retention of the C-O functionality in the coating. The process is terminated when the exterior film layer has reached the desired composition. The succession of thin layers, each differing slightly in composition in a progressive fashion from the preceding one, results in a significantly more adhesive composite coating composition bonded to the substrate than coatings deposited without adjusting the deposition conditions under a relatively lower plasma duty cycle.

Gas-phase depositions, particularly plasma depositions, provide coating effective amounts of coating or amounts sufficient to increase the hydrophilic characteristic of the surface. Typically, such coatings are of substantially uniform thickness and of an amount so that the thickness of the coating composition is about 5 Å and 5 μm, more preferably about 50 Å and 1 μm, and most preferably about 100 Å and 0.1 μm. The uniform film thickness and controllability of the deposition method may be contrasted with thickness controllability problems encountered using previously disclosed methods. Using the RF pulsed plasma deposition provides linearity of the thickness of the coating composition with deposition time for a given plasma duty cycle and fixed monomer pressure and flow rate.

The coatings of this invention increase the hydrophilic character of the surface, particularly of surfaces that are hydrophobic (e.g., polysiloxanes). The extent of hydrophilicity introduced during the plasma process was observed to increase as the oxygen content of the plasma generated coating compositions increased.

The surfaces with coating compositions of this invention are suited for contact lenses and other biomedical devices. The coating compositions exhibit good adhesion, high wettability, high oxygen permeability, and excellent transparency in the visible region of the electromagnetic spectrum when deposited on polymer surfaces. The adhesion of the coating compositions to these surfaces is sufficiently strong to resist delamination.

Thus the coating composition of this invention satisfies the stringent criteria listed above to improve the biocompatibility of contact lenses. The emphasis in this invention has been placed on the contact lenses; however, those skilled in the art wfll recognize that the highly wettable, biologically non-fouling, transparent coatings of this invention are useful for various other applications (e.g., other biomedical devices, biosensors, detectors deployed in marine environments, membranes, tissue culture growth, implants, etc.).

The invention will be clarified further by a consideration of the following, non-limiting examples.

Examples

Water contact angle measurements were measured using dynamic (modified Wilhelmy plate) methods for coated and uncoated surfaces. Dynamic measurements were made using surfaces immersed in succession in a saline solution. Advancing contact angles were measured under dynamic conditions. The dynamic measurements were each repeated four times as the sample was cycled up and down, with the average value being recorded for these four measurements.

Example 1

An air-dried silicone hydrogel contact lens was first treated with argon plasma in continuous wave mode at 100w for 5 minutes on each side. The argon plasma pressure was at 0.25 torr. The lens further was treated in ethylene glycol (Aldrich) plasma in a pulsing mode of 10 ms on and 200 ms off at 100w for 15 minutes on each side. The ethylene glycol vapor pressure was kept at 0.086 torr. The grafted lens was washed with saline solution and stored in saline solution for testing.

The advancing contact angles are shown on Table 1. The dynamic wetting studies reveal consistently lower contact angles for the coated surfaces with the surface wettability being appreciably better than the uncoated lens.

Overall, the water contact angle measurements illustrate the transformation of the initial hydrophobic polymer surface to a hydrophilic wettable surface as provided by the plasma deposited coatings.

TABLE 1

| Surface | Advancing Angle (deg.) |
|---|---|
| Uncoated Lens | 133 ∀ 8 |
| Coated Lens | 78 ∀ 4 |

Example 2

An air dried, silicone hydrogel contact lens first was treated with argon plasma in continuous wave mode at 100w for 5 minutes on each side. The argon pressure was 0.25 torr. The lens was treated in 1-vinyl-2-pyrrolidone (Lancaster) plasma in a pulsing mode of 10 ms on and 200 ms off at 100w for 15 minutes on each side. The 1-vinyl-2-pyrrolidone vapor pressure was kept at 0.08 torr. The grafted lens was washed with saline solution and stored in saline solution for testing. The results of dynamic contact angle testing are shown on Table 2.

TABLE 2

| Surface | Advancing Angle (deg.) |
|---|---|
| Uncoated Lens | 133 ∀ 8 |

TABLE 2 (continued)

| Surface | Advancing Angle (deg.) |
|---------|------------------------|
| Coated Lens | 66 ∀ 8 |

Example 3

An air dried, silicone hydrogel contact lens first was treated with argon plasma in continuous wave mode at 100w for 5 minutes on each side. The argon pressure was 0.25 torr. The lens then was treated in N, N'-dimethylacrylamide (Jarchem) plasma in a pulsing mode of 10 ms on and 200 ms off at 100w for 15 minutes on each side. The 1-vinyl-2-pyrrolidone vapor pressure was kept at 0.08 torr. The grafted lens was washed with saline solution and stored in saline solution for testing. The results of dynamic contact angle testing are shown on Table 3.

TABLE 3

| Surface | Advancing Angle (deg.) |
|---------|------------------------|
| Uncoated Lens | 133 ∀ 8 |
| Coated Lens | 68 ∀ 4 |

Example 4

An air-dried, silicone hydrogel contact lens first is treated with argon plasma in continuous wave mode on each side. The lens is treated in vinyl acetate plasma in a pulsing mode on each side. The grafted lens is washed with saline solution and stored in saline solution for testing. Similarly, silicone hydrogel lenses are surface grafted with vinyl acetic acid, acrylic acid, or 3,3-dimethacrylic acid using the same technique. The lenses show a significant wettability improvement over uncoated silicone hydrogel lenses.

**Claims**

1. A device comprising at least one surface having a coating effective amount of a coating composition, said coating composition being formed by the gas phase polymerization of a gas comprising at least one monomer consisting of vinyl pyrrolidone, N,N'-dimethylacrylamide, ethylene glycol, vinyl acetate, vinyl acetic acid, acrylic acid, 3,3-dimethylacrylic acid, or a mixture thereof.

2. The device of claim 1, which is a contact lens.

3. The device of claim 1 or claim 2, wherein the gas phase polymerization is pulsed having a duty cycle of less than about 1/5 in which the plasma on time is 10 μsec to 100 msec and the plasma off time is 100 μsec to 2000 msec.

4. The device of claim 1 or claim 2, wherein the gas phase polymerization is high voltage discharge, radio frequency, microwave, ionizing radiation induced plasma polymerization, photo induced polymerization or a combination thereof.

5. The device of any one of claims 1 to 4, wherein the coating composition is gradient layered by systematically decreasing the duty cycle of the gas phase polymerization.

6. A method for manufacturing devices comprising contacting at least one surface of a device with a coating effective amount of a coating composition, wherein the coating composition is formed by the gas phase polymerization of a gas comprising at least one monomer consisting of vinyl pyrrolidone, N,N'-dimethylacrylamide, ethylene glycol, vinyl acetate, vinyl acetic acid, acrylic acid, 3,3-dimethylacrylic acid, or a mixture thereof

7. The method of claim 6, wherein the device is a contact lens.

8. The method of claim 6 or claim 7, wherein the gas phase polymerization is pulsed having a duty cycle of less than about 1/5 in which the plasma on time is 10 μsec to 100 msec and the plasma off time is 100 μsec to 2000 msec.

9. The process of claim 6 or claim 7, wherein the gas phase polymerization is high voltage discharge, radio frequency, microwave, ionizing radiation induced plasma polymerization, photo induced polymerization or a combination thereof.

10. The process of any one of claims 6 to 10, wherein the coating composition is gradient layered by systematically decreasing the duty cycle of the gas phase polymerization.